# EUROPÄISCHE PATENTSCHRIFT

(11) **EP 0 724 868 B1**
(45) Veröffentlichungstag und Bekanntmachung des Hinweises auf die Patenterteilung: **27.06.2001**
(21) Anmeldenummer: 95810057.0
(22) Anmeldetag: 31.01.1995
(51) Int. Cl.: A61F 2/38

(54) **Gelenkprothese, insbesondere Kniegelenkprothese**
Joint prothesis, in particular a knee prosthesis
Prothèse d'articulation, notamment prothèse d'articulation du genou

(43) Veröffentlichungstag der Anmeldung: 07.08.1996
(73) Patentinhaber: Sulzer Orthopädie AG, 6340 Baar (CH)
(72) Erfinder: Frei, Heribert, CH-8200 Schaffhausen (CH)
(74) Vertreter: Sulzer Management AG

(56) Entgegenhaltungen:
- EP-A- 0 519 873
- DE-A- 2 636 816
- FR-A- 2 330 375
- FR-A- 2 696 927

## Beschreibung

Die Erfindung betrifft eine Gelenkprothese, insbesondere eine Kniegelenkprothese gemäss dem Oberbegriff von Anspruch 1.

Aus der EP 0 519 873 ist eine Gelenkprothese, insbesondere eine Kniegelenkprothese bekannt, welche ein Femurteil, ein Tibiateil und ein Zwischenteil aufweist. Das Femurteil besitzt einen Verankerungsabschnitt zur Befestigung im Femur und zwei Kondylen. Das Tibiateil besitzt einen Verankerungsabschnitt zur Befestigung in der Tibia. Das Zwischenteil liegt auf einer ebenen Gleitfläche des Tibiateils auf und weist zwei konkave Gleitlager zur Lagerung der Kondylen des Femurteils auf. Das Zwischenteil ist derart gelagert, dass es eine rotatorische Bewegung um eine Achse senkrecht zur Gleitfläche aufweist sowie eine translatorische Bewegung parallel zur Gleitfläche.
Diese bekannte Kniegelenkprothese lässt sich auch für Fälle verwenden, bei denen das hintere Kreuzband defekt ist oder bei denen weder das hintere Kreuzband noch die Seitenbänder erhalten geblieben sind. Ein möglicher Nachteil dieser bekannten Kniegelenkprothese könnte darin zu sehen sein, dass das Kniegelenk in den vorhin genannten Fällen eine ungenügende Stabilität in anterior-posterior Richtung oder in varus-valgus Richtung aufweist.

Es ist daher Aufgabe der vorliegenden Erfindung, die beschriebenen Nachteile der bekannten Prothese zu vermeiden. Insbesondere soll eine Prothese geschaffen werden, die sich für Fälle eignet, bei denen das hintere Kreuzband defekt ist oder bei denen weder das hintere Kreuzband noch die Seitenbänder erhalten geblieben sind. Es soll eine Prothese geschaffen werden, die in anterior-posterior Richtung als auch in varus-valgus Richtung eine genügende Stabilität aufweist.

Diese Aufgabe wird gelöst gemäss den Merkmalen von Anspruch 1. Die Unteransprüche 2 bis 14 beziehen sich auf weitere vorteilhafte Ausgestaltungen der Erfindung.

Die erfindungsgemässe Gelenkprothese umfasst mindestens ein erstes Prothesenteil, das einen Verankerungsabschnitt sowie mindestens einen Drehgelenkabschnitt aufweist, sowie ein zweites Prothesenteil, das einen Verankerungsabschnitt sowie eine Gleitfläche aufweist, sowie ein zwischen dem Drehgelenkabschnitt und der Gleitfläche angeordnetes Zwischenteil, wobei das zweite Prothesenteil sowie das Zwischenteil je ein Führungsteil umfasst, nämlich das zweite Prothesenteil ein erstes Führungsteil und das zwischenteil ein zweites Führungsteil, welche beiden Führungsteile derart zusammenwirken, dass das Zwischenteil bezüglich einer Bewegung in anteriorer/posteriorer Richtung geführt ist. Die erfindungsgemässe Gelenkprothese umfasst weiter ein nach proximal aus dem zweiten Prothesenteil vorstehendes drittes Führungsteil. Das erste Prothesenteil ist mit einem Kupplungsorgan verbunden, und das Kupplungsorgan weist zum in proximaler Richtung vorstehenden dritten Führungsteil eine Wirkverbindung auf, derart, dass das Kupplungsorgan entlang dem dritten Führungsteil verschiebbar ist. Das Kupplungsorgan ist so ausgebildet, dass es eine erste Drehachse definiert, um welche herum das erste Prothesenteil bei einer Flexions- bzw. Extensionsbewegung relativ zu dem zweiten Prothesenteil beugbar ist. Das dritte Führungsteil ist zylinderförmig ausgebildet und das Kupplungsorgan ist so ausgebildet, dass das Kupplungsorgan und damit die erste Drehachse relativ zu diesem dritten Führungsteil nur in proximaler bzw. distaler Richtung, nicht jedoch in anteriorer/posteriorer Richtung verschiebbar ist.

Gemäss einer bevorzugten Ausführungsform der Erfindung ist die Gelenkprothese als eine Kniegelenkprothese mit einem Femurteil, einem Tibiateil und einem Zwischenteil beziehungsweise einem Meniskusteil ausgebildet.

Die erfindungsgemässe Kniegelenkprothese weist ein Tibiateil mit einem im wesentlichen in proximaler Richtung vorstehenden Führungsteil auf. Das Femurteil ist schwenkbar mit einem Kupplungsorgan verbunden, und das Kupplungsorgan weist eine Wirkverbindung zum Führungsteil auf, derart, dass das Tibiateil und das Femurteil eine gegenseitige Verschiebbarkeit in Verlaufsrichtung des Führungsteiles aufweisen und dabei gegenseitig geführt sind. Das schwenkbar mit dem Femurteil verbundene Kupplungsorgan bestimmt bezüglich einer Flexion-Extension Bewegung die gemeinsame Drehachse zwischen dem Femurteil und dem Tibiateil. Weiter ist das Zwischenteil zumindest in einer translatorischen Richtung beweglich auf dem Tibiateil gelagert.

Ein Vorteil der erfindungsgemässen Prothese ist darin zu sehen, dass das Femurteil und das Tibiateil durch das Führungsteil in anterior-posterior Richtung gegenseitig geführt sind. Dadurch eignet sich die erfindungsgemässe Prothese als Implantat, wenn das hintere Kreuzband defekt ist, um eine anterior-posterior Stabilisierung des Kniegelenkes zu bewirken.

In einer weiteren vorteilhaften Ausführungsform der Kniegelenkprothese sind das Femurteil, das Führungsteil und das Kupplungsorgan zusammen mit dem Zwischenteil und dem Tibiateil derart gegenseitig ausgestaltet, dass eine gegenseitige Schwenkbewegung von Femurteil und Tibiateil in varus-valgus Richtung eingeschränkt oder verhindert wird. Eine derartige Kniegelenkprothese weist den Vorteil auf, dass sie nebst der anterior-posterior Stabilisierung auch eine varus-valgus Stabilisierung aufweist. Eine derart ausgeführte mit einer Stabilisierung in varus-valgus Richtung eignet sich insbesondere als Implantat, wenn sowohl die hinteren Kreuzbänder als auch die Seitenbänder defekt sind.

Das in proximaler Richtung vorstehende Führungsteil legt eine Rotationsachse fest, um welche das Kupplungsorgan schwenkbar gelagert ist, derart, dass das Femurteil und das Tibiateil um eine in proximaler Richtung verlaufende Achse gegenseitig verdrehbar sind. In einer weiteren vorteilhaften Ausführungsform ist die Kniegelenkprothese derart ausgestaltet, dass sie eine Rotationshemmung bezüglich der erwähnten gegenseitigen Verdrehung aufweist, sodass das Femurteil und das Tibiateil ab einem durch die Konstruktion vorgegebenen Winkelbetrag gegen Verdrehung progressiv gehemmt wird. Eine Kniegelenkprothese mit einer derart ausgestaltete Rotationshemmung eignet sich insbesondere wenn sowohl die hinteren Kreuzbänder als auch die Seitenbänder defekt sind.

Das Femurteil weist Drehgelenkabschnitte auf, welche auf verschiedenartige Weise ausgebildet sein können, um bezüglich einer Flexions-Extensions Bewegung eine Drehbewegung zum Tibiateil zu ermöglichen. Eine vorteilhafte Ausführungsform besteht darin, dass der Drehgelenkabschnitt des Femurteils durch zwei Kondylen gebildet ist, und dass das Zwischenteil zwei entsprechend dem Verlauf der Drehgelenkabschnitte ausgestaltete Lagerflächen zur Zusammenarbeit mit den Kondylen des Femurteils aufweist. Es erweist sich als vorteilhaft, den Drehgelenkabschnitt des Femurteils mit einer derartigen Führungsbahn auszugestalten, dass die Führungsbahn unterschiedliche Radien, insbesondere einen von anterior nach posterior kleiner werdenden Radius aufweist. Um zwischen den Flächen des Zwischenteils und dem Drehgelenkabschnitt des Femurteils bezüglich einer Flexions-Extensions Bewegung immer eine hohe Kongruenz beizubehalten kann es sich jedoch auch als vorteilhaft erweisen, die Führungsbahn mit einem konstanten Krümmungsradius auszugestalten.

Das Tibiateil kann derart ausgestaltet sein, dass die Beweglichkeit des Zwischenteils relativ zum Tibiateil auf Bewegungen in einer Gleitbahnebene eingeschränkt ist. Die Gleitfläche des Tibiateils kann jedoch auch eine Wölbung aufweisen, sodass das Zwischenteil auf einer gekrümmten Fläche auf dem Tibiateil gleitet.

Gemäss einer bevorzugten Ausführungsform der Erfindung ist die Gelenkprothese als eine Kniegelenkprothese mit einem Femurteil, einem Tibiateil und einem Zwischenteil beziehungsweise einem Meniskusteil ausgebildet. Weitere Einzelheiten der Erfindung ergeben sich aus der nachfolgenden Beschreibung von Kniegelenkprothesen unter Bezugnahme auf die Zeichnungen, in denen einige bevorzugte Ausführungsformen beispielsweise veranschaulicht sind.

Es zeigt:
- Fig. 1a: eine Explosionsdarstellung eines ersten Ausführungsbeispieles einer Prothese, z.B. einer Kniegelenkprothese;
- Fig. 1b: eine Explosionsdarstellung ein zweites Ausführungsbeispiel einer Kniegelenkprothese;
- Fig. 1c: ein weiteres Ausführungsbeispiel eines Tibiateils;
- Fig. 2a: eine Seitenansicht ein drittes Ausführungsbeispiel einer Kniegelenkprothese bei Extension;
- Fig. 2b: eine Seitenansicht des dritten Ausführungsbeispieles gemäss Fig. 2a bei Flexion;
- Fig. 3a: einen Längsschnitt (A-A) durch das dritte Ausführungsbeispiel gemäss Fig. 2a;
- Fig. 3b: einen Längsschnitt durch ein viertes Ausführungsbeispiel einer Kniegelenkprothese ;
- Fig. 4a: eine weitere Seitenansicht des ersten Ausführungsbeispieles gemäss Fig. la;
- Fig. 4b: eine Seitenansicht des ersten Ausführungsbeispieles gemäss Fig. la;
- Fig. 5a: einen Schnitt durch ein viertes Ausführungsbeispiel einer Kniegelenkprothese;
- Fig. 5b: ein weiterer Schnitt durch das vierte Ausführungsbeispiel gemäss Fig. 5a;
- Fig. 5c: eine Aufsicht auf ein Tibiateil mit einem Zwischenteil des vierten Ausführungsbeispieles einer Kniegelenkprothese;
- Fig. 5d: eine Aufsicht auf das Tibiateil mit einer weiteren Ausführungsform eines Zwischenteils;
- Fig. 6a: einen Schnitt durch ein fünftes Ausführungsbeispiel einer Kniegelenkprothese;
- Fig. 6b: ein weiterer Schnitt durch das fünftes Ausführungsbeispiel gemäss Fig. 6a;
- Fig. 7: einen Schnitt durch ein sechstes Ausführungsbeispiel einer Kniegelenkprothese.

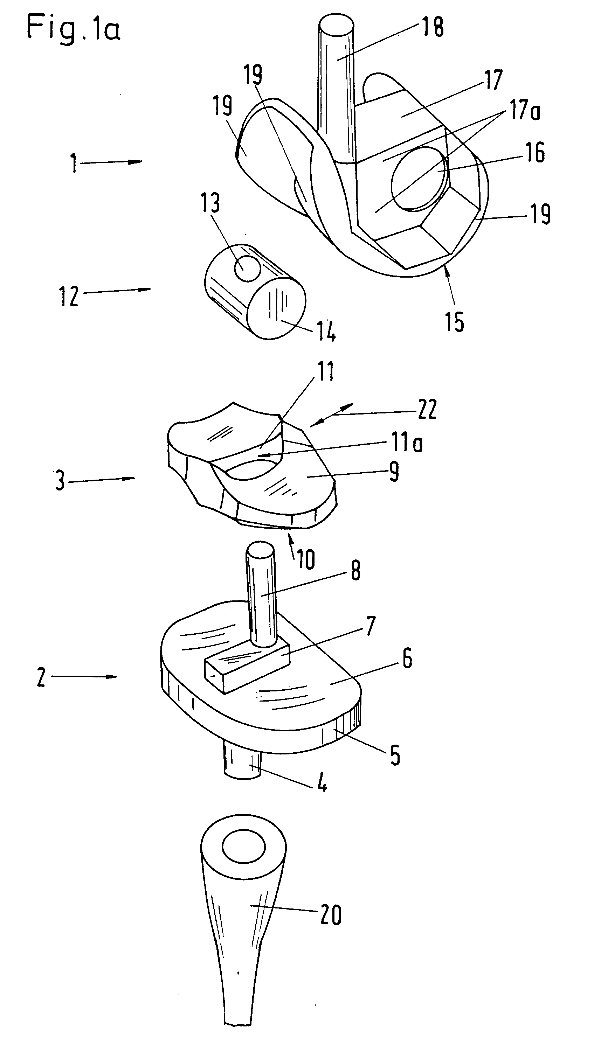

Fig. 1a zeigt ein erstes Ausführungsbeispiel einer Kniegelenkprothese. Das Femurteil 1 weist einen Verankerungsabschnitt 18 zur Verankerung im Femur auf, sowie zwei Kondylen 19, wobei die kondylenartig sphärisch gewölbten Flächen einen Drehgelenkabschnitt 15 bilden. Das Femurteil 1 weist weiter ein Stabilisierungsorgan 17 mit Seitenwandungen 17a auf sowie eine die Seitenwandung 17a durchbrechende zylinderförmige Ausnehmung 16. Das Tibiateil 2 weist eine Platte 5 auf, deren eine Oberfläche als eine ebene Gleitfläche 6 ausgestaltet ist, wobei aus der Gleitfläche 6 ein länglich ausgestaltetes Führungsteil 7 vorsteht, welches fest mit der Platte 5 verbunden ist und zur Führung eines Meniskus- oder Zwischenteils 3 dient. Das Tibiateil 2 weist weiter ein senkrecht oder annähernd senkrecht zur Gleitfläche 6 vorstehendes, zylinderförmiges Führungsteil 8 auf, das in proximaler Richtung verläuft. Weiter weist das Tibiateil 2 auf der dem Führungsteil 7 abgewandten Seite einen Verankerungsabschnitt 4 auf, der dazu dient, das Tibiateil 2 in der Tibia 20 zu verankern. Das Zwischenteil 3 ist als ein Meniskusteil ausgestaltet, das zwischen die Gleitfläche 6 und der Kondyle 19 zu liegen kommt, wobei das Zwischenteil 3 eine entsprechend dem Verlauf des Drehgelenkabschnittes 15 angepasste konkave Lagerfläche 9 aufweist sowie auf der Rückseite eine entsprechend der Gleitfläche 6 angepasste ebene Gleitfläche 10. Zudem weist das Zwischenteil 3 eine Ausnehmung 11 mit einer Führungsbahn lla auf, wobei das Führungsteil 7 in die Ausnehmung 11 einfügbar ist, sodass die Ausnehmung 11 zusammen mit dem Führungsteil 7 die Beweglichkeit des Zwischenteils 3 bezüglich dem Tibiateil 2 festlegt. In dem dargestellten ersten Ausführungsbeispiel ist das Zwischenteil 3 in der durch die Gleitfläche 6 definierten Ebene in einer durch den Verlauf des Führungsteils 7 vorgegebenen translatorischen Richtung 22 hin und her beweglich. Weiter ist ein Kupplungsorgan 12 dargestellt, das als ein zylinderförmiges Teil 14 mit einer zylinderförmigen Ausnehmung 13 ausgestaltet ist.

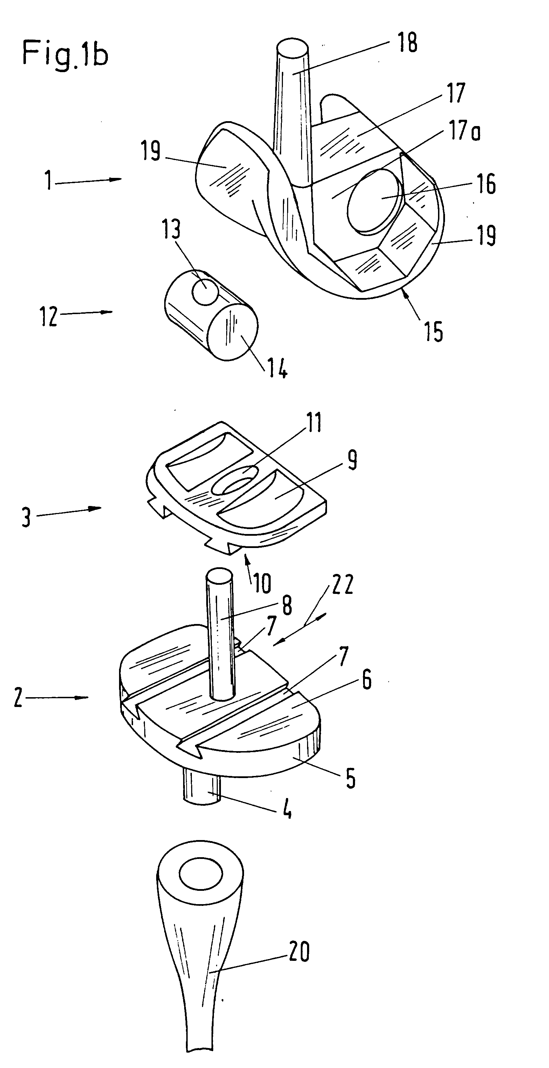

Fig. 1b zeigt ein zweites Ausführungsbeispiel einer Kniegelenkprothese, welche ähnlich dem Ausführungsbeispiel gemäss Fig. la ausgestaltet ist, mit dem Unterschied, dass die Gleitfläche 6 schwalbenschwanzförmig ausgebildete, geradlinig verlaufende Nuten aufweist, die als Führungsteil 7 dienen, um das Zwischenteil 3 in translatorischer Richtung 22 beweglich zu lagern. Das Zwischenteil 3 weist seinerseits nebst der Ausnehmung 11 und der konkaven Lagerfläche 9 ein entsprechend angepasstes Führungsteil lla zur Aufnahme des Führungsteils 7 auf.

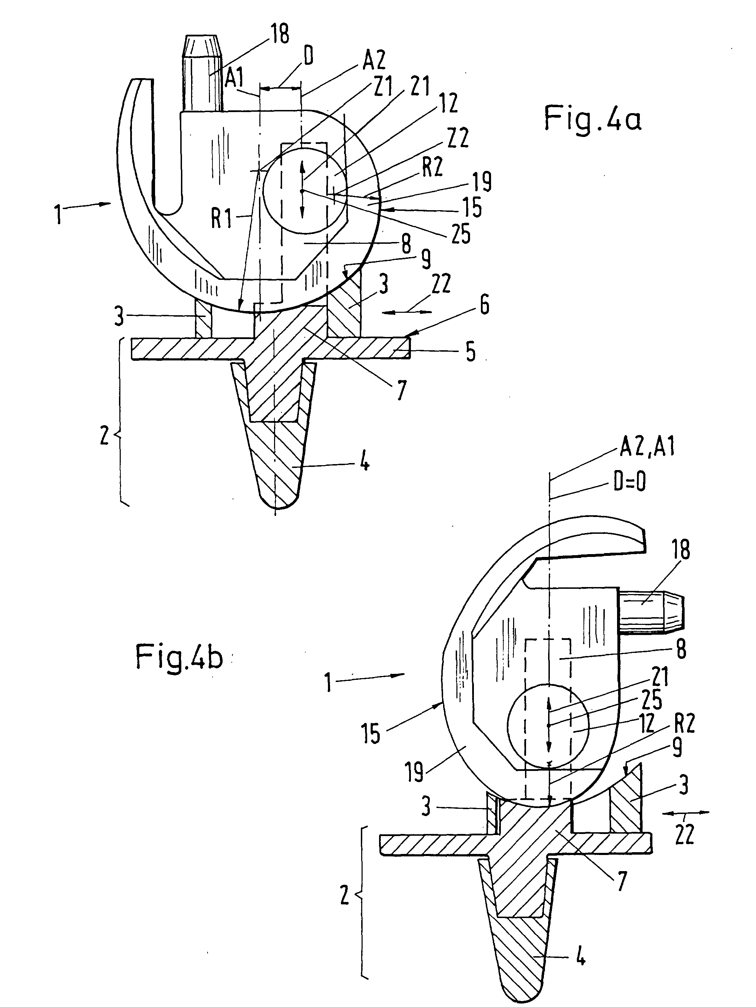

Fig. 4a zeigt eine Seitenansicht des ersten Ausführungsbeispieles gemäss Fig. la in zusammengesetztem Zustand. Die Kniegelenkprothese ist in einer Stellung der Extension dargestellt. Das Femurteil 1 ist in einer Seitenansicht dargestellt und zeigt einen Drehgelenkabschnitt 15 mit variierendem Krümmungsradius, wobei das Femurteil 1 mit einem Krümmungsradius R1 auf dem Zwischenteil 3 aufliegt, und wobei sich der Krümmungsradius R in Richtung zunehmender Flexion auf einen Radius R2 verringert. Die Radien R1, R2 haben unterschiedliche Krümmungszentren Z1, Z2. Das innerhalb des Femurteils 1 verlaufende Führungsteil 8 ist strichliert dargestellt, wobei das Führungsteil 8 durch die zylinderförmige Ausnehmung 13 des Kupplungsorganes 12 verläuft und dadurch eine Wirkverbindung zwischen dem Femurteil 1 und dem Tibiateil 2 herstellt. Das Kupplungsorgan 12 definiert eine Drehachse 25, um welche das Femurteil 1 sowie das Tibiateil 2 bezüglich einer Extension-Flexion Bewegung gegenseitig drehbar gelagert sind. Das Kupplungsorgan 12 bzw. das Femurteil 1 ist in Richtung 21 gegenüber dem Führungsteil 8 bzw. dem Tibiateil 2 verschiebbar. Das geradlinig verlaufende Führungsteil 8 weise eine Mittenachse A2 auf. Das Femurteil 1 ist über das Kupplungsorgan 12 um diese Achse A2 drehbar mit dem Führungsteil 8 verbunden, sodass das Femurteil 1 gegenüber dem Tibiateil 2 sowie dem Zwischenteil 3 um die Achse A2 drehbar gelagert ist. Das Zwischenteil 3 sowie das Tibiateil 2 sind in einem Schnitt dargestellt, wobei das Führungsteil 7 sowie das Führungsteil 8 fest mit der Platte 5 verbunden sind. Das Zwischenteil 3 ist in Bewegungsrichtung 22 durch das Führungsteil 7 in translatorischer Richtung geführt verschiebbar, wobei die maximale Auslenkung durch ein Anschlagen des Zwischenteil 3 an das Führungsteil 7 begrenzt wird.

In der dargestellen Lage einer Extension weist der Drehgelenkabschnitt 15 einen Krümmungsradius R1 auf, wobei der Verlauf des Drehgelenkabschnittes 15 sowie der Verlauf der konkaven Lagerfläche 9 des Zwischenteils 3 derart gegenseitig angepasst, dass die beiden Flächen eine hohe Kongruenz aufweisen, sodass eine Flächenberührung mit einer entsprechend geringen Flächenbelastung resultiert.

Fig. 4b zeigt die bereits in Fig. 4a dargestellte Kniegelenkprothese in einer Flexionsstellung. Der auf dem Zwischenteil 3 aufliegende Drehgelenkabschnitt 15 weist einen kleineren Krümmungsradius R2 auf, sodass sich zwischen dem Zwischenteil 3 und dem Femurteil 1 eine kleine Berührungsfläche oder gar eine linienförmige Berührung ergibt. Das Femurteil 1 sowie das Tibiateil 2 können aufgrund der Wirkverbindung zwischen Kupplungsorgan 12 und Führungsteil 8 keine gegenseitige Relativbewegung in anterior-posterior Richtung ausführen, dafür ist eine gegenseitige Relativbewegung entlang der durch das Führungsteil 8 festgelegten Achse A2 in Verlaufsrichtung 21 möglich. Das Zwischenteil 3 ist in Bewegungsrichtung 22 translatorisch verschiebbar, was zum Beispiel bei einer Extension-Flexion Bewegung den Vorteil hat, dass das Zwischenteil 3 zumindest bei belasteter Kniegelenkprothese von selbst eine Lage mit einer hohen Kongruenz zwischen den Flächen 9, 15 zu erlangen sucht. In einer vorteilhaften Ausgestaltung wird der Verlauf der beiden Flächen 9, 15 derart gegenseitig angepasst, dass sich in einem ersten Flexionsbereich, der von einer Senkrechten zur Gleitfläche 6 ( Lage der Extension) bis zu einem Winkel von etwa 53 Grad bezüglich der Senkrechten verläuft, eine hohe Kongruenz zwischen dem Drehgelenkabschnitt 15 des Femurteils 1 und der Lagerfläche 9 des Zwischenteils 3 ergibt. Bei einer über 53 Grad hinausgehenden Flexion ergibt sich eine zunehmend kleinere Berührungsfläche, die bis zu einer linienförmigen Berührung übergehen kann.

Das Femurteil 1 sowie das Tibiateil 2 sind um eine durch den Verlauf des Führungsteiles 8 festgelegte Drehachse A2, die in proximaler Richtung verläuft, gegenseitig verdrehbar. Die Kniegelenkprothese ist in der in Fig. 4a dargestellten Lage der Extension üblicherweise durch eine entgegengesetzt der proximalen Richtung wirkenden Kraft stark belastet, wobei sich auf Grund der Flächenberührung zwischen dem Femurteil 1 und dem Zwischenteil 3 eine kleine Flächenbelastung ergibt. Auf Grund der Flächenberührung und der gegenseitigen Ausgestaltung von Femurteil 1 und Zwischenteil 3 ergibt sich eine Drehachse A1, um welche die beiden Teile bei abwesendem Führungsteil 8 drehbar wären. Sowohl die grosse einwirkende Kraft als auch die ausgeprägte Flächenberührung würden in der Lage der Extension ein Verdrehen des Femurteils 1 gegenüber dem Tibiateil 2 um die Drehachse A1 erschweren. Das Führungsteil 8 legt die Achse A2 fest, um welche das Femurteil 1 und das Tibiateil 2 gegenseitig verdrehbar sind, wobei sich zwischen den beiden Drehachsen A1, A2 ein Abstand D ergibt. Diese geometrische Anordnung der Drehachsen A1, A2 in der Lage der Extension verhindert ein gegenseitiges Verdrehen zwischen Femurteil 1 und Tibiateil 2. Eine derartige Ausführung einer Kniegelenkprothese lässt somit bei gestrecktem und belastetem Bein keine Rotation zu.

Im Gegensatz dazu lässt die Kniegelenkprothese bei Flexion und insbesondere bei gleichzeitig unbelastetem Bein eine Rotation zu, wie dies im weiteren mit Fig. 4b gezeigt wird. In der Lage der Flexion liegt der Drehgelenkabschnitt 15 nur linienförmig auf der konkaven Lagerfläche 9 auf, wobei die von der Kniegelenkprothese zu übertragenden Kraft bei Flexion gering ist, sodass die Flächenbelastung gering ausfällt. Die geringe gegenseitige Berührungsfläche in Verbindung mit der geringen übertragenen Kraft bewirken, dass das Femurteil 1 und das Tibiateil 2 leicht und somit durch geringe Kräften um die Drehachse A2 gegenseitig verdrehbar sind. Vorteilhafterweise werden die gegenseitigen Berührungsflächen zudem derart ausgestaltet, dass sich die bei Flexion ergebende Drehachse A1 mit der Lage der durch das Führungsteil 8 vorgegebenen Drehachse A2 zusammenfällt und somit die Distanz D zwischen den Drehachsen A1, A2 zu Null wird, was zusätzlich ein Verdrehen von Femurteil 1 und Tibiateil 2 erleichtert.

Somit weist das erste Ausführungsbeispiel der Kniegelenkprothese ein Verhalten auf, das bei Flexion keine Rotation zwischen Femurteil 1 und Tibiateil 2 ermöglicht, wogegen bei Flexion ein gewisses Mass an Rotation möglich ist. Dadurch eignet sich das erste Ausführungsbeispiel insbesondere als Implantat, wenn sowohl die hinteren Kreuzbänder als auch die Seitenbändern nicht mehr vorhanden sind.

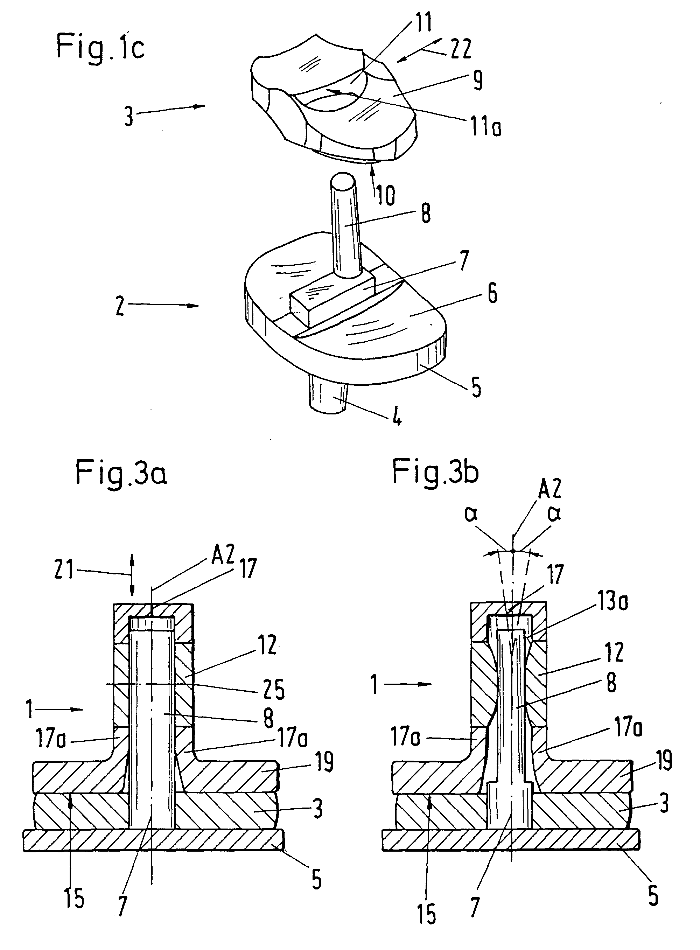

Fig. 1c zeigt eine weitere Ausgestaltung eines Tibiateiles 2, welches eine konkav ausgestaltete Gleitfläche 6 aufweist. Das Zwischenteil 3 weist eine entsprechend ausgestaltete Gleitfläche 10 auf, sodass das Zwischenteil 3 in der gewölbten Gleichfläche 6 in einer durch das Führungselement 7 bestimmten, translatorischen Richtung gleitbar gelagert ist.

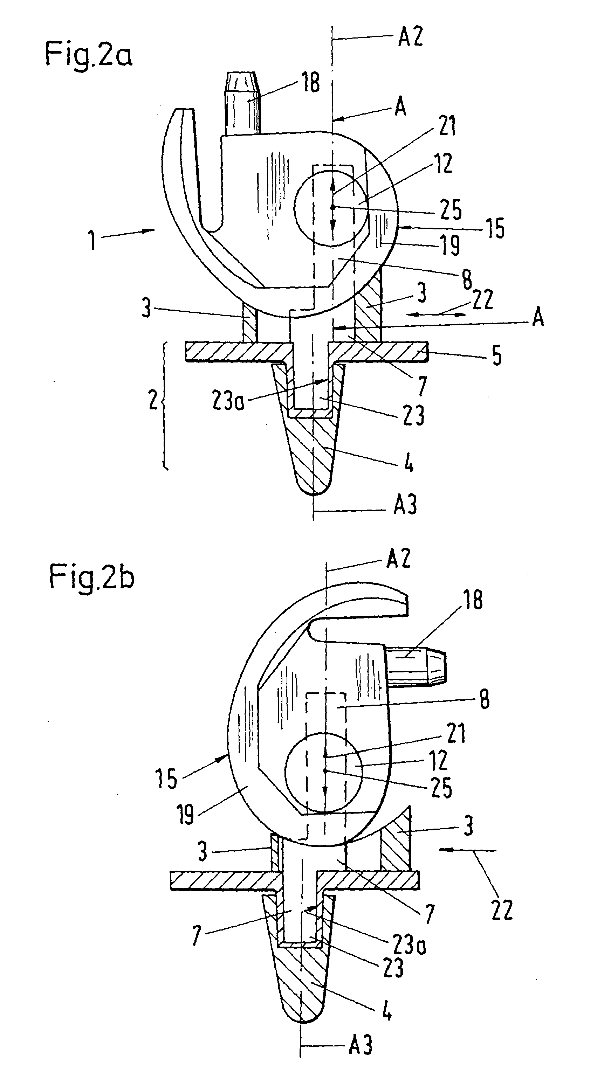

Das in Fig. 2a dargestellte dritte Ausführungsbeispiel einer Kniegelenkprothese in Extension weist ein Führungsteil 8 auf, das, wie zum Beispiel in Fig. la dargestellt senkrecht zur Gleitfläche 6 verlaufend angeordnet und mit dem Führungsteil 7 verbunden ist. Das in Fig. 2a dargestellte dritte Ausführungsbeispiel weist gegenüber der in Fig. la dargestellten Ausführung den Unterschied auf, dass das Führungsteil 7 drehbar zur Gleitfläche 6 mit dieser verbunden ist. Fig. 2a zeigt einen Zapfen 23, der drehbar in einer Ausnehmung des Tibiateils 2 gelagert ist, wobei die Ausnehmung senkrecht zur Gleitfläche 6 verläuft. Das Führungsteil 7 ist fest mit dem Zapfen 23 verbunden und dadurch bezüglich Platte 5 drehbar gelagert. Weiter ist das Führungsteil 8 mit dem Führungsteil 7 verbunden, sodass auch das Führungsteil 8 bezüglich der Platte 5 drehbar gelagert ist. Das Führungsteil 7 ist wiederum derart ausgestaltet, dass das Zwischenteil 3 translatorisch zu einer durch die Stellung des Führungsteils 7 bestimmten Verlaufsrichtung verschiebbar ist. Der Zapfen 23 definiert eine Achse A3, um die der Zapfen 23 mit Führungsteil 7 und Führungsteil 8 drehbar gelagert sind. Somit ist das Femurteil 1 und das Tibiateil 2 sowohl in Flexion wie auch in Extension gegenseitig um die Achse A3 drehbar. Das dritte Ausführungsbeispiel eignet sich insbesondere als Implantat, wenn die hinteren Kreuzbänder nicht mehr vorhanden sind, die Seitenbänder jedoch noch vorhanden sind. Im dargestellten Ausführungsbeispiel gemäss Fig. 2a ist das Führungsteil 8 zylinderförmig ausgebildet, sodass das Femurteil 1 zudem um die durch das Führungsteil 8 definierte Drehachse A2 gegenüber dem Tibiateil 2 verdrehbar ist. Das Führungsteil 8 kann jedoch zusammen mit dem Kupplungsorgan 12 auch derart ausgestaltet sein, dass bezüglich dem Führungsteil 8 und dem Femurteil 1 keine gegenseitige Verdrehung um eine Achse A2 möglich ist, zum Beispiel dadurch, dass das Führungsteil 8 und die Ausnehmung 13 im Kupplungsorgan 12 elliptisch ausgestaltet sind, sodass eine gegenseitige Verdrehung von Führungsteil 8 und Kupplungsorgan 12 nicht möglich ist.

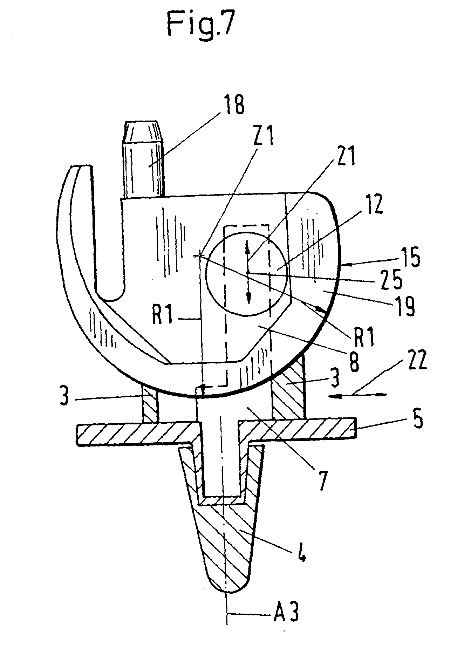

Fig. 7 zeigt ein sechstes Ausführungsbeispiel einer Kniegelenkprothese. Dieses Ausführungsbeispiel weist gegenüber dem dritten Ausführungsbeispiel gemäss Fig. 2a den Unterschied auf, dass der Drehgelenkabschnitt, welcher im Bereich zwischen Flexion und Extension mit dem Zwischenteil in Berührung kommt, einen konstanten Krümmungsradius R1 aufweist. Zudem weist die konkave Lagerfläche 9 des Zwischenteils 3 denselben Krümmungsradius R1 auf. Dadurch ergibt sich in jeder Stellung der Kniegelenkprothese zwischen Flexion und Extension eine hohe Kongruenz zwischen den sich berührenden Flächen mit einer entsprechend geringen Flächenbelastung, was einen geringen Verschleiss zur Folge hat.

Fig. 3a zeigt einen Längsschnitt (A-A) entlang dem Führungsteil 8 des dritten Ausführungsbeispiel gemäss Fig. 2a. Die Kondyle 19 liegt mit dem Drehgelenkabschnitt 15 auf dem Zwischenteil 3 auf. Das Führungsteil 8 sowie des daran anschliessende Führungsteil 7 sind über einen nicht dargestellten Zapfen 23 drehbar in der Platte 5 gelagert. Das Stabilisierungsorgan 17, 17a des Femurteils 1 ist um eine Drehachse 25 drehbar mit dem Kupplungsorgan 12 verbunden. Weiter ist das Kupplungsorgan 12 in einer durch die Drehachse A2 vorgegebenen Richtung 21 verschiebbar. In Fig. 3b ist ein viertes Ausführungsbeispiel einer Kniegelenkprothese dargestellt, mit einem wie in Fig. 3a verlaufenden Schnitt, jedoch mit dem Unterschied, dass das Führungsteil 8 einen kleineren Durchmesser aufweist, als die Breite des Innenraumes des Stabilisierungsorganes 17, 17a. Zudem weist das Kupplungsorgan 12 eine sich gegen aussen erweiternde Öffnung 13a auf. Dadurch ist das Femurteil 1 gegenüber dem Führungsteil 8 in varus-valgus Richtung um einen Winkel a schwenkbar. Die Auslenkung in varus-valgus Richtung ist durch die Anschlagteile 17a begrenzt. Die Ausführung gemäss Fig. 3a verhindert eine Auslenkung in varus-valgus Richtung, da die Anschlagteile 17a unmittelbar auf dem Führungsteil 8 aufliegen.

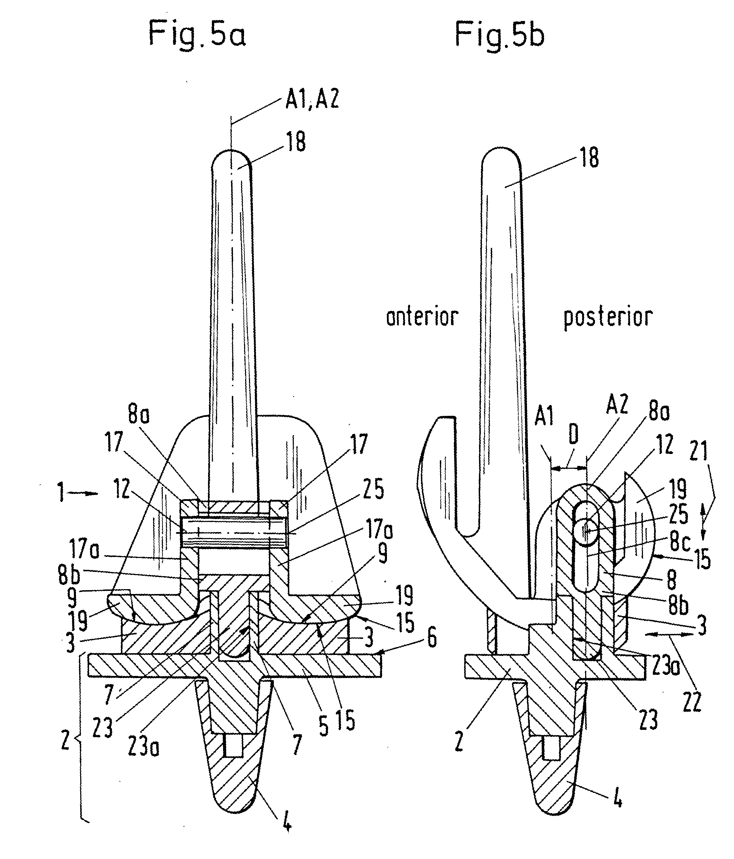

Die Figuren 5a und 5b zeigt je einen Längsschnitt aus unterschiedlichen Blickrichtungen durch ein viertes Ausführungsbeispiel einer Kniegelenkprothese. Das Femurteil 1 umfasst eine Kondyle 19 mit einem Drehgelenkabschnitt 15, ein mit einem Kupplungsorgan 12 verbundenes Stabilisierungsorgan 17 sowie einem Verankerungsabschnitt 18. Das Tibiateil 2 umfasst einen Verankerungsabschnitt 4, eine Platte 5 sowie ein mit der Platte fest verbundenes Führungsteil 7, das ein Zwischenteil 3 in einer translatorischen Bewegungsrichtung 22 führt. Ein Führungsteil 8 ist mit einem Zapfen 23 drehbar um eine Achse A2 im Führungsteil 7 gelagert und verläuft senkrecht zu der durch die Platte 5 aufgespannten Gleitfläche 6. Das Führungsteil 8 weist eine in Richtung der Drehachse A2 verlaufende Ausnehmung 8c auf, in welcher das Kupplungsorgan 12 geführt ist, sodass das Kupplungsorgan 12 um eine Drehachse 25 drehbar und in Verlaufsrichtung der Drehachse A2 verschiebbar ist. Die Ausnehmung 8c ist in proximaler Richtung durch einen oberen Begrenzungsanschlag 8a begrenzt, sodass die Drehachse 25 maximal bis zum Begrenzungsanschlag 8a verschiebbar ist. Im diesem vierten Ausführungsbeispiel verläuft die Drehachse 25 entlang der Drehachse A2. Das Führungsteil weist beidseitig eine annähernd spielfreie Wirkverbindung zum Stabilisierungsorgan 17, 17a auf, sodass eine Schwenkbewegung zwischen Femurteil 1 und Tibiateil 2 in varus-valgus Richtung nicht möglich ist. Weiter ist die bereits mit Fig. 4a beschriebene Drehachse A1 dargestellt.

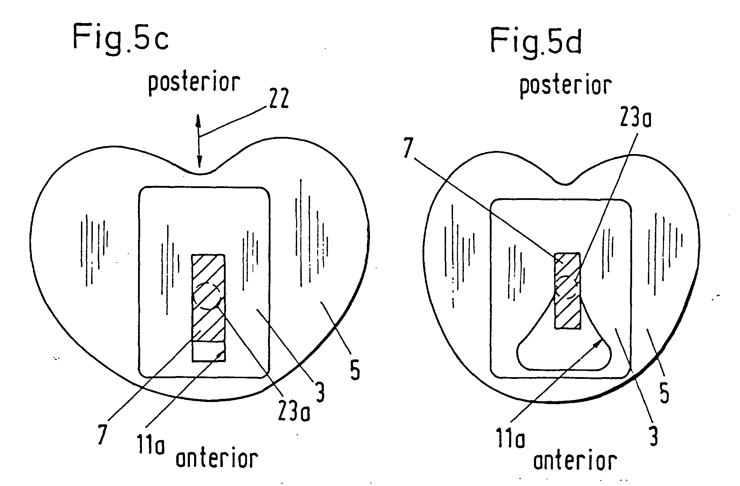

Fig. 5c zeigt eine Aufsicht auf das Tibiateil 2 mit Platte 5, Führungsteil 7 und Ausnehmung 23a zur Halterung des Zapfens 23, sowie ein um das Führungsteil 7 gelegtes Zwischenteil 3, das durch das Führungsteil 7 in translatorischer Richtung 22 geführt ist. Das Zwischenteil 3 ist in einer Stellung bei Extension der Kniegelenkprothese dargestellt. Fig. 5d zeigt dieselbe Anordnung wie Fig. 5c jedoch mit einem Zwischenteil 3, das in anteriorer Richtung eine Erweiterung 11a aufweist. Das Zwischenteil 3 wird bei einer Beugung des Kniegelenkprothese von Extension nach Flexion in posteriorer Richtung verschoben, sodass das Zwischenteil 3 um die Drehachse A2 drehbar gelagert ist, wobei das Spiel des Zwischenteils 3 mit zunehmender Flexion, das heisst mit zunehmender Verschiebung in posteriorer Richtung zunimmt, sodass das Femurteil 1 mit zunehmender Flexion leichter um die Drehachse A2 beziehungsweise um das Tibiateil 2 drehbar ist. Weiter ergibt sich die bereits mit Fig. 4a beschriebene Anordnung der beiden Drehachsen A1, A2, die bei Extension um eine Distanz D beabstandet sind, und die sich mit zunehmender Beugung gegenseitig annähern, sodass auch auf Grund dieses Effektes das Femurteil 1 zunehmend leichter um die Drehachse A2 beziehungsweise um das Tibiateil 2 drehbar ist.

Eine Kniegelenkprothese gemäss dem vierten Ausführungsbeispiel und einem Zwischenteil gemäss Fig. 5c oder Fig. 5d lässt bei gestrecktem und belastetem Bein keine Rotation zwischen Femurteil 1 und Tibiateil 2 um die Drehachse A2 zu, wogegen bei Flexion ein gewisses Mass an Rotation um die Achse A2 möglich ist. Dadurch eignet sich das vierte Ausführungsbeispiel insbesondere als Implantat, wenn sowohl die hinteren Kreuzbänder als auch die Seitenbändern nicht mehr vorhanden sind.

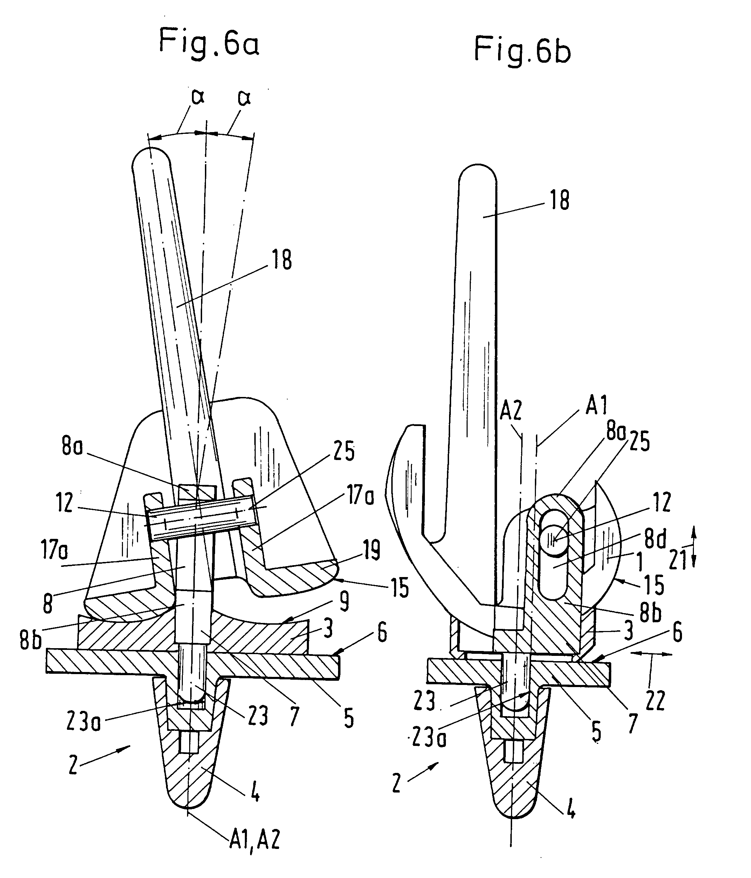

Die Figuren 6a und 6b zeigen je einen Längsschnitt aus unterschiedlichen Blickrichtungen durch ein fünftes Ausführungsbeispiel einer Kniegelenkprothese. Aus Fig. 6a ist ersichtlich, dass dieses Ausführungsbeispiel eine Auslenkung zwischen Femurteil 1 und Tibiateil 2 in varus-valgus Richtung zulässt. Im Vergleich zu Fig. 5a ist das Führungsteil 8 wesentlich schmaler ausgestaltet, sodass das Femurteil 1 durch das Führungsteil 8 derart gehalten ist, dass der Verankerungsabschnitt 18 in varus-valgus Richtung um einen Winkel α zur Drehachse A2 schwenkbar gelagert ist. Der Winkel α ist einerseits begrenzt durch die Anschlagteile 17a, die ab einer gewissen Auslenkung am Führungsteil 8 anstehen, andererseits wirkt der obere Begrenzungsanschlag 8a des Führungsteils 8 begrenzend, sobald das Kupplungsorgan 12, wie in Fig. 6a dargestellt, den Begrenzungsanschlag 8a berührt. Der maximal schwenkbare Winkel α lässt sich durch eine entsprechende geometrische Ausgestaltung der den Winkel α beeiflussenden Teile konstruktiv auf einfache Weise festlegen. Die in Fig. 6a dargestellte Anordnung zur Auslenkung zwischen dem Femurteil 1 und dem Tibiateil 2 in varus-valgus Richtung zulässt sich auch im vierten Ausführungsbeispiel gemäss Fig. 5a anwenden, sodass dieses vierte Ausführungsbeispiel eine begrenzte Auslenkung in varus-valgus Richtung aufweisen würde.

Fig. 6a und 6b weist ein Tibiateil 2 mit einer senkrecht zur Gleitfläche 6 verlaufenden, zylinderförmigen Ausnehmung 23a auf, welche als Lager für einen drehbaren Zapfen 23 dient, der eine Drehachse A2 festlegt. Das Führungsteil 8 weist eine länglich ausgestaltete Ausnehmung 8d auf, entlang welcher das Kupplungsorgan 12 in Verlaufsrichtung der Drehachse A2 beweglich gelagert ist, wobei die Ausnehmung 8d in proximaler Richtung einen oberen Begrenzungsanschlag 8a aufweist. Das Führungsteil 8 ist fest mit dem Führungsteil 7 verbunden. Das Führungsteil 7 dient zur Führung des Zwischenteils 3 und ist als rechteckiger Körper ausgestaltet, um das Zwischenteil 3 bezüglich dem Führungsteil 7 in translatorischer Richtung 22 zu führen. Das Führungsteil 7 ist fest mit dem Drehzapfen 23 verbunden, sodass das Führungsteil 7 zusammen mit dem Führungsteil 8 um die Drehachse A2 drehbar gelagert ist.

Weiter ergibt sich, wie bereits mit Fig. 4a beschrieben, eine zweite Drehachse A1, die sich durch das Femurteil 1 sowie das Zwischenteil 3 zusammen mit dem Tibiateil 2 ergibt. Wie in Fig. 6b dargestellt, weisen die beiden Drehachsen A1, A2 bei Extension eine geringen Abstand D auf, der sich mit zunehmender Flexion vergrössert, indem, bezogen auf die Darstellung gemäss Fig. 6b, die Drehachse A1 nach rechts verschoben wird.

Das fünfte Ausführungsbeispiel eignet sich insbesondere als Implantat, wenn die hinteren Kreuzbänder nicht mehr vorhanden sind, die Seitenbänder jedoch noch vorhanden sind.

Das Femurteil 1, das Tibiateil 2, das Kupplungsorgan 12 sowie das Führungsteil 8 bestehen vorteilhaft aus einem Metall oder einer Metallegierung, wie sie üblicherweise für Gelenkprothesen verwendet wird. Das Zwischenteil 3 kann aus einem Kunststoff gefertigt sein, als auch aus einer Metallegierung bestehen, weil auf Grund der teilweise kongruenten Flächen die Flächenpressung und somit auch der Reibungsverschleiss klein gehalten werden kann. Möglich ist auch eine Verwendung von Keramik für alle Teile, wobei aber eine Metallegierung vorzuziehen ist, weil sie duktiler ist.

## Patentansprüche

1. Gelenkprothese, insbesondere Kniegelenkprothese, mit mindestens einem ersten Prothesenteil (1), das einen Verankerungsabschnitt (18) sowie mindestens einen Drehgelenkabschnitt (15) aufweist, einem zweiten Prothesenteil (2), das einen Verankerungsabschnitt (4) sowie eine Gleitfläche (6) aufweist, und einem zwischen dem Drehgelenkabschnitt (15) und der Gleitfläche (6) angeordneten Zwischenteil (3), wobei das zweite Prothesenteil (2) sowie das Zwischenteil (3) je ein Führungsteil (7, 11a) umfassen, nämlich das zweite Prothesenteil (2) ein erstes Führungsteil (7) und das Zwischenteil (3) ein zweites Führungsteil (11a), wobei das erste Führungsteil (7) und das zweite Führungsteil (11a) derart zusammenwirken, dass das Zwischenteil (3) bezüglich einer Bewegung in anteriorer/posteriorer Richtung geführt ist, wobei ferner das zweite Prothesenteil (2) ein nach proximal vorstehendes drittes Führungsteil (8) umfasst und wobei mit dem ersten Prothesenteil (1) ein Kupplungsorgan (12) verbunden ist, welches zu diesem dritten Führungsteil (8) eine Wirkverbindung aufweist, derart, dass das Kupplungsorgan (12) entlang dem dritten Führungsteil (8) verschiebbar ist, **dadurch gekennzeichnet**, dass das Kupplungsorgan (12) so ausgebildet ist, dass es eine erste Drehachse (25) definiert, um welche herum das erste Prothesenteil (1) bei einer Flexions- bzw. Extensionsbewegung relativ zu dem zweiten Prothesenteil (2) beugbar ist, dass das dritte Führungsteil (8) zylinderförmig ausgebildet ist und das Kupplungsorgan (12) so ausgebildet ist, dass das Kupplungsorgan (12) und damit die este Drehachse (25) relativ zu diesem dritten Führungsteil (8) nur in proximaler bzw. distaler Richtung, nicht jedoch in anteriorer/posteriorer Richtung verschiebbar ist.

2. Gelenkprothese nach Anspruch 1, dadurch gekennzeichnet, dass das erste Führungsteil (7) und/oder das dritte Führungsteil (8) fest mit dem zweiten Prothesenteil (2) verbunden ist.

3. Gelenkprothese nach einem der Ansprüche 1 bis 2, dadurch gekennzeichnet, dass die Gleitfläche (6) eben oder gewölbt ausgestaltet ist.

4. Gelenkprothese nach einem der vorangehenden Ansprüche, dadurch gekennzeichnet, dass das erste Führungsteil (7) oder das dritte Führungsteil (8) einen Drehzapfen (23) aufweist, dass der Drehzapfen (23) in einer Bohrung (23a) gelagert ist, die sich in das zweite Prothesenteil (2) erstreckt, sodass eine zweite Drehachse (A3) festgelegt wird, um welche das erste Führungsteil (7) und/oder das dritte Führungsteil (8) drehbar gelagert ist.

5. Gelenkprothese nach einem der Ansprüche 1 bis 4, dadurch gekennzeichnet, dass das dritte Führungsteil (8) in proximaler Richtung geradlinig verlaufend ausgestaltet ist, um das Kupplungsorgan (12) bei einer gegenseitigen Verschiebung der Prothesenteile (1,2) geradlinig zu führen.

6. Gelenkprothese nach einem der Ansprüche 1 bis 5, dadurch gekennzeichnet, dass das Führungsteil (8) kreiszylinderförmig ausgestaltet ist und eine in proximaler Richtung weisende dritte Drehachse (A2) definiert, um welche herum das Kupplungsorgan (12) drehbar gelagert ist.

7. Gelenkprothese nach einem der Ansprüche 1 bis 6, dadurch gekennzeichnet, dass das dritte Führungsteil (8) einen Anschlag (8a,8b) aufweist, um den Weg der Verschiebung des Kupplungsorganes (12) beziehungsweise die gegenseitige Verschiebung der Prothesenteile (1,2) in distaler und/oder proximaler Richtung zu begrenzen.

8. Gelenkprothese nach einem der Ansprüche 1 bis 7, dadurch gekennzeichnet, dass das erste Führungsteil (7) und das zweite Führungsteil (11a) derart ausgestaltet sind, dass das zweite Führungsteil (11a) in anteriorer Richtung ein zunehmendes Spiel relativ zum ersten Führungsteil (7) aufweist, sodass ein zu einer posterioren Stellung hin verschobenes Zwischenteil (3) gegenüber dem zweiten Prothesenteil (2) eine zunehmende rotatorische Beweglichkeit aufweist.

9. Gelenkprothese nach einem der Ansprüche 1 bis 8, dadurch gekennzeichnet, dass das erste Prothesenteil (1) ein in proximaler Richtung sich erstreckendes Stabilisierungsteil (17) mit mindestens zwei Begrenzungsteilen (17a) umfasst, dass das dritte Führungsteil (8) zwischen den Begrenzungsteilen (17a) verlaufend angeordnet ist, und dass die Begrenzungsteile (17a) bei einer gegenseitigen Relativbewegung der Prothesenteile (1,2) in varus-valgus Richtung den maximalen Neigungswinkel (α) begrenzen.

10. Gelenkprothese nach Anspruch 9, dadurch gekennzeichnet, dass das dritte Führungsteil (8) annähernd spielfrei zwischen den Begrenzungsteilen (17a) verlaufend angeordnet ist, um keine gegenseitige Relativbewegung der Prothesenteile (1,2) in varus-valgus Richtung zuzulassen.

11. Gelenkprothese nach einem der Ansprüche 1 bis 10, dadurch gekennzeichnet, dass der Drehgelenkabschnitt (15) eine gewölbte Fläche aufweist mit einem ersten Krümmungsradius (R2) bei 90 Grad Flexion und einem zweiten, grösseren Krümmungsradius (R1) in Extension.

12. Gelenkprothese nach einem der Ansprüche 1 bis 10, dadurch gekennzeichnet, dass der Drehgelenkabschnitt (15) eine gewölbte Fläche mit einem konstanten Krümmungsradius (R1) aufweist.

13. Gelenkprothese nach einem der Ansprüche 11 oder 12, dadurch gekennzeichnet, dass das Zwischenteil (3) eine Lagerfläche (9) für den Drehgelenkabschnitt (15) aufweist, und dass die Lagerflächen (9) und der Drehgelenkabschnitt (15) derart gegenseitig angepasst sind, dass sie bei Extension der Gelenkprothese eine hohe Kongruenz aufweisen.

14. Gelenkprothese nach einem der Ansprüche 1 bis 13, dadurch gekennzeichnet, dass das Femurteil (1), das Tibiateil (2), das Kupplungsorgan (12) sowie das erste Führungsteil (7) und/oder das dritte Führungsteil (8) aus einem Metall oder einer Metallegierung gefertigt sind, und dass das Zwischenteil (3) aus einem Kunststoff, einer Keramik, einem Metall oder einer Metallegierung gefertigt ist.

## Claims

1. A joint prosthesis, in particular a knee joint prosthesis, having at least a first prosthesis part (1) which comprises an anchoring portion (18) as well as at least one pivot joint portion (15); a second prosthesis part (2) comprising an anchoring portion (4) as well as a slide surface (6); and an intermediate piece (3) arranged between the pivot joint portion (15) and the slide surface (6), wherein the second prosthesis part (2) as well as the intermediate part (3) each include a respective guide part (7, 1 la), that is the second prosthesis part (2) includes a first guide part (7) and the intermediate part (3) includes a second guide part (11a), with the first guide part (7) and the second guide part (11a) cooperating such that the intermediate part (3) is guided with respect to a movement in an anterior/posterior direction, and furthermore with the second prosthesis (2) comprising a third guide part (8) projecting in a proximal direction and the first prosthesis part (1) being connected to a coupling member (12) having an operative connection to said third guide part (8) such that the coupling member (12) is displaceable along said third guide part (8), characterised in that the coupling member (12) is designed such that it defines a first axis of rotation (25) around which the first prosthesis part (1) can be deflected relative to the second prosthesis part (2) in a flexion movement or an extension movement, in that the third guide part (8) is formed in a cylindrical shape and the coupling member (12) is formed such that the coupling member (12) and thus the first axis of rotation (25) is displaceable only in a proximal or distal direction relative to said third guide part (8), but not in an anterior/posterior direction.

2. A joint prosthesis in accordance with claim 1, characterised in that the guide part (7) and/or the guide part (8) is fixedly connected to the second prosthesis part (2).

3. A joint prosthesis in accordance with one of the claims 1 to 2, characterised in that the slide surface (6) is formed planar or arched.

4. A joint prosthesis in accordance with one of the preceding claims, characterised in that the guide part (7) or the guide part (8) has a pivot pin (23) and in that the pivot pin (23) is journalled in a bore (23a) extending into the second prosthesis part (2) so that an axis of rotation (A3) is defined about which the guide part (7) and/or the guide part (8) are rotatably journalled.

5. A joint prosthesis in accordance with one of the claims 1 to 4, characterised in that the guide part (8) is formed so as to extend in the proximal direction in a straight line to guide the coupling member (12) in a straight line on relative displacement of the prosthesis parts (1, 2).

6. A joint prosthesis in accordance with one of the claims 1 to 5, characterised in that the guide part (8) is formed in a cylindrical shape and defines an axis of rotation (A2) pointing in its direction of extension and about which the coupling member (12) is rotatably journalled.

7. A joint prosthesis in accordance with one of the claims 1 to 6, characterised in that the third guide part (8) has an abutment (8a, 8b) in order to limit the displacement path of the coupling member (12) i.e. to limit the relative displacement of the prosthesis parts (1, 2) in the distal direction and/or the proximal direction.

8. A joint prosthesis in accordance with one of the claims 1 to 7, characterised in that the first guide part (7) and the second guide part (11a) are designed such that the second guide part (11a) has increasing play relative to the first guide part (7) in an anterior direction so that an intermediate part (3) displaced towards the posterior position has increasing rotational mobility with respect to the second prosthesis part (2).

9. A joint prosthesis in accordance with one of the claims 1 to 8, characterised in that the first prosthesis part (1) includes a stabiliser part (17) extending in the proximal direction and having at least two bounding parts (17a); in that the guide part (8) is arranged extending between the bounding parts (17a); and in that the bounding parts (17a) limit the maximum angle of inclination (à) in the varus-valgus direction for a mutual relative movement of the prosthesis parts (1, 2).

10. A joint prosthesis in accordance with claim 9, characterised in that the third guide part (8) is arranged substantially free of play extending between the bounding parts (17a) in order to permit no mutual relative movement of the prosthesis parts (1, 2) in the varus-valgus direction.

11. A joint prosthesis in accordance with one of the claims 1 to 10, characterised in that the pivot joint portion (15) has an arched surface with a first radius of curvature (R2) at 90 degrees of flexion and a second, larger radius of curvature (R1) in extension.

12. A joint prosthesis in accordance with one of the claims 1 to 10, characterised in that the pivot joint portion (15) has an arched surface with a constant radius of curvature (R1).

13. A joint prosthesis in accordance with one of the claims 11 or 12, characterised in that the intermediate part (3) has a bearing surface (9) for the pivot joint portion (15); and in that the bearing surfaces (9) and the pivot joint portion (15) are matched to one another such that they display a high degree of congruency on extension of the joint prosthesis.

14. A joint prosthesis in accordance with one of the claims 1 to 13, characterised in that the femur part (1), the tibia part (2), the coupling member (12) as well as the first guide part (7) and/or the third guide part (8) are fabricated from a metal or a metal alloy; and in that the intermediate part (3) is fabricated from a plastic, a ceramic, a metal or a metal alloy.

## Revendications

1. Prothèse d'articulation, notamment prothèse d'articulation du genou, avec au moins une première partie de prothèse (1) qui présente un tronçon d'ancrage (18) et au moins un tronçon d'articulation tournant (15), une deuxième partie de prothèse (2) qui présente un tronçon d'ancrage (4) ainsi qu'une face de glissement (6), et une partie intermédiaire (3) disposée entre le tronçon d'articulation tournant (15) et la face de glissement (6), où la deuxième partie de prothèse (2) ainsi que la partie intermédiaire (3) comprennent chacune une partie de guidage (7, 11a), à savoir la deuxième partie de prothèse (2) une première partie de guidage (7) et la partie intermédiaire (3) une deuxième partie de guidage (11a), où la première partie de guidage (7) et la deuxième partie de guidage (11a) coopèrent de façon que la partie intermédiaire (3) soit guidée relativement à un mouvement dans la direction antérieure/postérieure, où en outre la deuxième partie de prothèse (2) comprend une troisième partie de guidage (8) faisant saillie proximalement et où il est relié à la première partie de prothèse (1) -un organe d'accouplement (12) qui est en liaison active avec cette troisième partie de guidage (8) de façon que l'organe d'accouplement (12) soit déplaçable le long de la troisième partie de guidage (8), caractérisée en ce que l'organe de couplage (12) est réalisé de façon à définir un premier axe de rotation (25) autour duquel peut être fléchie la première partie de prothèse (1) lors d'un mouvement de flexion respectivement d'extension relativement à la deuxième partie de prothèse (2), en ce que la troisième partie de guidage (8) est réalisée en une forme cylindrique et en ce que l'organe de couplage (12) est réalisé de telle sorte que l'organe de couplage (12) et par conséquent le premier axe de rotation (25) est déplaçable relativement à cette troisième partie de guidage (8) seulement dans la direction proximale respectivement distale, mais non pas dans la direction antérieure/postérieure.

2. Prothèse d'articulation selon la revendication 1, caractérisée en ce que la première partie de guidage (7) et/ou la troisième partie de guidage (8) sont reliées solidement à la deuxième partie de prothèse (2).

3. Prothèse d'articulation selon l'une des revendications 1 à 2, caractérisée en ce que la face de glissement (6) est réalisée en une forme plane ou bombée.

4. Prothèse d'articulation selon l'une des revendications précédentes, caractérisée en ce que la première partie de guidage (7) ou la troisième partie de guidage (8) présente un pivot (23), en ce que le pivot (23) est logé dans un perçage (23a) qui s'étend dans la deuxième partie de prothèse (2) de telle sorte qu'un deuxième axe de rotation (A3) est déterminé autour duquel est logée d'une manière pivotante la première partie de guidage (7) et/ou la troisième partie de guidage (8).

5. Prothèse d'articulation selon l'une des revendications 1 à 4, caractérisée en ce que la troisième partie de guidage (8) est réalisée avec une extension rectiligne dans la direction proximale pour guider en ligne droite l'organe d'accouplement (12) lors d'un déplacement mutuel des parties de prothèse (1, 2).

6. Prothèse d'articulation selon l'une des revendications 1 à 5, caractérisée en ce que la partie de guidage (8) est réalisée en une forme cylindrique circulaire, et qu'il est défini un troisième axe de rotation (A2) orienté dans la direction proximale autour duquel est logé d'une manière tournante l'organe d'accouplement (12).

7. Prothèse d'articulation selon l'une des revendications 1 à 6, caractérisée en ce que la troisième partie de guidage (8) présente une butée (8a, 8b) pour limiter le trajet de déplacement de l'organe d'accouplement (12) respectivement le déplacement mutuel des parties de prothèse (1, 2) dans la direction distale et/ou proximale.

8. Prothèse d'articulation selon l'une des revendications 1 à 7, caractérisée en ce que la première partie de guidage (7) et la deuxième partie de guidage (11a) sont réalisées de façon que la deuxième partie de guidage (11a) présente dans la direction antérieure un jeu croissant relativement à la première partie de guidage (7) de telle sorte qu'une partie intermédiaire (3) déplacée vers une position postérieure présente par rapport à la deuxième partie de prothèse (2) une mobilité de rotation croissante.

9. Prothèse d'articulation selon l'une des revendications 1 à 8, caractérisée en ce que la première partie de prothèse (1) comprend une partie de stabilisation (17) s'étendant dans la direction proximale avec au moins deux parties de limitation (17a), en ce que la troisième partie de guidage (8) est disposée pour s'étendre entre les parties de limitation (17a) et que les parties de limitation (17a), lors d'un mouvement relatif mutuel des parties de prothèse (1, 2) dans la direction varus-valgus, limitent l'angle d'inclinaison maximal (α).

10. Prothèse d'articulation selon la revendication 9, caractérisée en ce que la troisième partie de guidage (8) est disposée approximativement sans jeu entre les parties de limitation (17a) pour ne pas permettre de mouvement relatif mutuel des parties de prothèse (1, 2) dans la direction varus-valgus.

11. Prothèse d'articulation selon l'une des revendications 1 à 10, caractérisée en ce que le tronçon d'articulation tournant (15) présente une face bombée avec un premier rayon de courbure (R2) à 90° de flexion et un deuxième rayon de courbure plus grand (R1) en extension.

12. Prothèse d'articulation selon l'une des revendications 1 à 10, caractérisée en ce que le tronçon d'articulation tournant (15) présente une face bombée avec un rayon de courbure constant (R1).

13. Prothèse d'articulation selon l'une des revendications 11 ou 12, caractérisée en ce que la partie intermédiaire (3) présente une face de palier (9) pour le tronçon d'articulation tournant (15), et en ce que les faces de palier (9) et le tronçon d'articulation tournant (15) sont adaptés mutuellement de façon à présenter une congruence élevée lors d'une extension de la prothèse d'articulation.

14. Prothèse d'articulation selon l'une des revendications 1 à 13, caractérisée en ce que la partie de fémur (1), la partie de tibia (2), l'organe d'accouplement (12) ainsi que la première partie de guidage (7) et/ou la troisième partie de guidage (8) sont fabriqués en un métal ou un alliage métallique, et en ce que la partie intermédiaire (3) est fabriquée en une manière synthétique, une céramique, un métal ou un alliage métallique.
